Europäisches Patentamt

European Patent Office

Office européen des brevets

(19)

(11) Veröffentlichungsnummer: **0 008 460**
**B1**

(12) **EUROPÄISCHE PATENTSCHRIFT**

(45) Veröffentlichungstag der Patentschrift: **19.05.82**

(21) Anmeldenummer: **79103087.7**

(22) Anmeldetag: **22.08.79**

(51) Int. Cl.³: **A 61 B 5/14,** G 01 N 27/40

(54) **Klebehalterurg und Verwendung derselben.**

(30) Priorität: **23.08.78 DE 2836904**

(43) Veröffentlichungstag der Anmeldung:
**05.03.80 Patentblatt 80/5**

(45) Bekanntmachung des Hinweises auf die Patenterteilung:
**19.05.82 Patentblatt 82/20**

(84) Benannte Vertragsstaaten:
**AT BE CH FR GB IT**

(56) Entgegenhaltungen:
**EP - A - 0 002 059**
**DE - A - 2 255 879**
**FR - A - 2 400 879**
**DE - A - 2 724 461**
**US - A - 4 041 932**

(73) Patentinhaber: **SIEMENS AKTIENGESELLSCHAFT**
**Berlin und München**
**Postfach 22 02 61**
**D-8000 München 22 (DE)**

(72) Erfinder: **Reichenberger, Helmut, Dr. Dipl.-Phys.**
**Irisstrasse 8**
**D-8501 Brand/Eckental (DE)**
Erfinder: **Zachmann, Wilfried**
**Nöttinger Strasse 41**
**D-7537 Remchingen (DE)**

Courier Press, Leamington Spa, England.

Klebehalterung und Verwendung derselben

Die Erfindung bezieht sich auf eine Klebehalterung für Meßwertaufnehmer, die eine Membran benötigen, insbesondere transkutane Sauerstoffelektroden, mit einem Klebeträger, der beidseitig wenigstens teilweise Klebeflächen aufweist, zum Ankleben an die Applikations-fläche des Aufnehmers einerseits und zum Ankleben des Klebeträgers samt Aufnehmer am Meßort andererseits. Unter Meßweraufnehmern sollen dabei neben transkutanen Sauerstoffelektroden im weitesten Sinne alle Aufnehmer verstanden werden, die in irgendeiner Form zur Erfassung von physiologischen Größen dienen. Darüber hinaus bezieht sich die Erfindung auf die Verwendung einer Klebehalterung speziell zur Applikation eines Aufnehmers zur transkutanen Messung des Sauerstoffpartialdrucks.

Zur Fixierung von transkutanen Sauerstoffelektroden am Meßort lassen sich im allgemeinen solche Kleberinge verwenden, die auch zur schnellen Applikation von EKG-Elektroden benutzt werden. Die Handhabung von transkutanen Sauerstoffelektroden zur störungsfreien Meßwertaufnahme ist jedoch ungleich aufwendiger als die normaler EKG-Elektroden. Die aus wenigstens einer Meßelektrode (Kathode) und gegebenenfalls einer weiteren Elektrode (Anode) bestehende Sauerstoffelektrode muß elektrodenseitig zunächst mit einem Elektrolyten versehen und mittels einer sauerstoffdurchlässigen Membran, deren Dicke üblicherweise zwischen 6 und 30 $\mu$m liegt, dicht abgedeckt werden. Dadurch wird eine komplette polarographische Meßzelle gebildet. Es hat sich aber gezeigt, daß es für die Patienten-Langzeitüberwachung mittels Sauerstoffelektroden unumgänglich ist, Elektrolyt und/oder Membran schnell und bequem austauschbar auf dem Aufnehmer vorzusehen. Dabei wird die Membran im allgemeinen mittels eines großflächigen Spannringes gehalten, der dann gleichzeitig als Auflagefläche für die Klebehalterung zwecks Fixieren am Meßort dient.

Vom Stand der Technik sind austauschbare Membranen für Sauerstoffelektroden bekannt: In der DE—A—26 28 288 wird beispielsweise vorgeschlagen, eine napf-, topf- oder schalenförmig vorgeformte Membran direkt über den als Gegenform ausgebildeten Meßwertaufnehmer zu schieben. Dabei wird dann kein separater Spannring mehr benötigt, der als Auflagefläche für eine Klebehalterung dienen könnte. Die Handhabung solcher Membranen ist diffizil und zeitaufwendig; sie erfordert besondere manuelle Geschicklichkeit, damit zwischen Membran und Elektrodenfläche im Elektrolyten keine Luftblasen gebildet werden. Zudem muß das Fixieren des gesamten Aufnehmers anschließend mittels Klebeband erfolgen. Bei einer derartigen Applikation besteht jedoch die Gefahr eines zu starken Andruckes an das Gewebe und dadurch einer Meßwertverfälschung.

Weiterhin ist aus der DE—A—27 24 461 eine elektrochemische Meßvorrichtung für die Messung durch die Haut hindurch vorbekannt, welche eine Membran benötigt, bei der neben dem eigentlichen Meßwertaufnehmer ein separates, hohlzylinderförmiges Montageteil vorhanden ist, das mit dem Meßwertaufnehmer verbunden werden kann. Bei dieser Meßvorrichtung ist die Membran in üblicher Weise am eigentlichen Meßwertaufnehmer angeordnet, wobei durch Vorschaltung des Montageteils ein zusätzlicher Raum zur Aufnahme von Elektrolyten od . dgl. gebildet ist. Die aus Meßwertaufnehmern einschließlich Membran und Montageteil jeweils montierte Meßvorrichtung kann mittels üblicher, von der EKG-Applikationstechnik bekannter, beidseitig klebender Laschen am Applikationsort fixiert werden. Alternativ dazu soll die Fixierung der gesamten Meßvorrichtung durch Ansaugen mittels einer Unterdruckquelle möglich sein.

Spezielle Vorteile von Klebehalterungen gegenüber letzterem Ansaugprinzip werden in der DE—A—27 24 461 nicht angegeben. Insgesamt sind bei Austausch der Meßvorrichtung mit dem Meßwertaufnehmer, Membran, Montageteil sowie gegebenenfalls Klebering eine Vielzahl von Einzelkomponenten vorhanden, deren korrekte Handhabung für eine sichere Applikation und Meßwerterfassung gewährleistet sein muß.

Aufgabe der Erfindung ist es, eine Klebehalterung zu schaffen, mit Meßwertaufnehmer, die eine Membran benötigen, leicht und sicher handhabbar und am Meßort applizierbar sind. Dabei soll einerseits die gegen Artefakte unempfindliche Fixierung des gesamten Aufnehmers und andererseits auch der Austausch bzw. Ersatz von Elektrolyt und Membran ohne großen Aufwand möglich sein.

Die Aufgabe ist erfindungsgemäß dadurch gelöst, daß der Klebeträger als Ring aus zwei aufeinanderliegenden, je beidseitig klebenden Folien gebildet wird, zwischen denen die Membran derart angeordnet ist, daß die Membran die Ringausnehmung im Klebeträger abdeckt und selbst einen Teil der Klebehalterung für den Meßwertaufnehmer bildet. Eine solche Klebehalterung in Weiterbildung von für EKG-Elektroden verwendbaren Kleberingen ist vorzugsweise mit je einem, vor unmittelbarem Gebrauch abziehbaren Schutzpapier abgedeckt. Neben der Klebefläche kann durch die Schutzpapiere gleichzeitig die empfindliche Membran geschützt werden. In weiterer zweckmäßiger Ausbildung kann nun auch zwischen Membran und dem Schutzpapier, das die auf den Aufnehmer aufklebbare Klebefläche abdeckt, schon bei Herstellung ein Elektrolyt eingeschlossen sein. Als Membran werden solche Materialien verwendet, die sauerstoffdurchlässig sind und eine gewisse Formelastizität aufweisen, beispielsweise Polypropylen, Polyäthylen, Polytetrafluoroäthylen od . dgl.

Besonders vorteilhaft bei der erfindungsgemäßen Klebehalterung ist die Verwendung dieser Klebehalterung zum Befestigen einer transkutanen Sauerstoffelektrode am Meßort. Es entfällt dabei praktisch ein Arbeitsgang, nämlich das separate Fixieren der Membran an den Elektroden. Bei der erfindungsgemäßen Klebehalterung wird lediglich das obere Schutzpapier von der Klebehalterung abgezogen, die Klebehalterung auf die ringartige Applikationsfläche der Sauerstoffelektrode von einer Seite beginnend aufgelegt und die Membran an die mit Elektrolyten versehenen Elektroden angedrückt. Durch solches Andrücken ist ein dichter Abschluß der Elektroden ohne Einschluß von Luftblasen od . dgl. gewährleistet. Wird als Sauerstoffelektrode eine solche mit leicht konvexer Ausbildung der Elektrodengrundfläche verwendet, so ergibt sich wegen der Formelastizität der Membran gleichzeitig eine gewisse Vorspannung der angedrückten Membran nach Ankleben der Klebehalterung.

In weiteren vorteilhaften Ausbildungen der Erfindung weist die Klebehalterung wenigstens eine Anfaßlasche auf. Mit dieser—nicht klebenden—Anfaßlasche kann nach Beendigung der Messung die Meßzelle vom Applikationsort leicht entfernt werden. Da bei Verwendung eines Klebeträgers aus zwei aufeinanderliegenden, je beidseitig klebenden Folien dementsprechend je Folie eine Anfaßlasche vorhanden ist, kann nach Abnahme der Sauerstoffelektrode vom Meßort auch die untere Klebefolie von dem Aufnehmer abgezogen werden. Die obere Folie mit Membran bleibt in diesem Fall also an der Sauerstoffelektrode fixiert; durch Aufkleben eines neuen, üblichen Kleberinges wird die Sauerstoffelektrode zur erneuten Applikation vorbereitet.

Weitere Einzelheiten und Vorteile der Erfindung ergeben sich aus der nachfolgenden Figurenbeschreibung von Ausführungsbeispielen anhand der Zeichnung in Verbindung mit den weiteren Unteransprüchen.

Es zeigen:

Fig. 1 eine Draufsicht einer erfindungsgemäßen Klebehalterung,

Fig. 2 einen Längsschmitt entlang der Linie II der Fig. 1,

Fig. 3 einen Längsschnitt durch eine Fig. 1 entsprechende Klebehalterung mit Abwandlungen und

Fig. 4 eine Sauerstoffelektrode, die mit der erfindungsgemäßen Klebehalterung am Meßort appliziert ist.

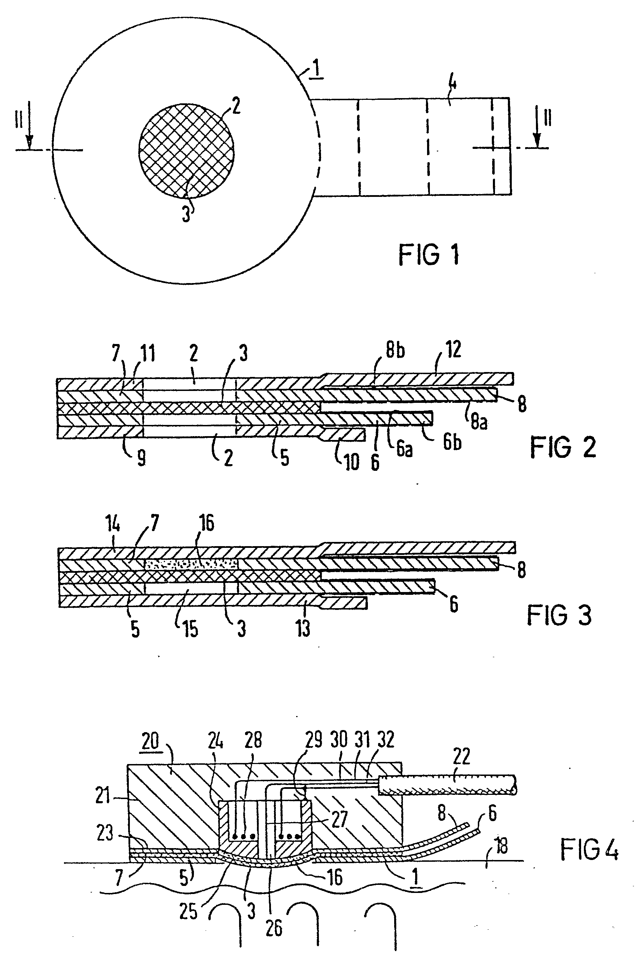

In der Fig. 1 ist mit 1 ein kreisförmiger Klebeträger bezeichnet, der speziell als Ring mit innerer Ausnehmung 2 ausgebildet ist. In der Ausnehmung 2 ist eine Membran 3 eingefügt. Die Größe des Ringes 1 ist auf die Auflagefläche einer Sauerstoffelektrode angepaßt, was in Fig. 4 im einzelnen erläutert wird. An dem Ring 1 ist eine Lasche 4 angeordnet, die zur Handhabung bei Ankleben der Klebehalterung an den Aufnehmer bzw. bei Entfernen des Aufnehmers vom Applikationsort dient.

In der Fig. 1 sind schematisch nur die Umrisse einer solchen Klebehalterung erkennbar. Die Struktur der erfindungsgemäßen Klebehalterung geht im einzelnen aus der Fig. 2 hervor, in der ein Schnitt längs der Linie II der Fig. 1 dargestellt ist. Mit 3 ist hier wiederum die schon aus Fig. 1 erkennbare sauerstoffdurchlässige Membran bezeichnet. Die Membran 3 weist den gleichen Durchmesser wie der Klebering 1 auf und ist zwischen zwei beidseitig klebende Folien 5 und 7 angeordnet. Die beiden Klebefolien 5 und 7 sind ringförmig mit einer Ausnehmung 2 ausgestanzt und mit rechteckförmigen Laschen 6 bzw. 8 versehen. Die beiden Laschen 6 bzw. 8 sind selbst mit ihren Oberflächen 6a, 6b und 8a, 8b nicht klebefähig. Auf die so aufgebaute Klebehalterung sind von jeder Seite weiterhin noch Schutzpapiere 9 bzw. 11 aufgebracht, die ebenfalls die kreisförmige Ausnehmung 2 aufweisen und entsprechende Anfaßlaschen 10 und 12 haben. Diese Anfaßlaschen liegen gerade auf den nicht klebefähigen Teilen der Folien 5 und 7 und vereinfachen damit das Abziehen der Schutzpapiere 9 und 11.

Insgesamt ergibt sich also bei einem derartigen Aufbau ein einheitlich großer Ring mitvon unten nach oben—weiter ausgreifenden Laschen, was für die Handhabung besonders vorteilhaft ist.

Die in der Fig. 3 dargestellte Klebehalterung entspricht in ihrem Aufbau weitgehend der Fig. 2. Die identischen Teile sind mit den gleichen Bezugszeichen wie die Fig. 1 und 2 numeriert. Allerdings sind in diesem Fall die Schutzpapiere 13 und 14 nicht mit Ausnehmungen ausgebildet. Es wird so vor Anwendung der Klebehalterung ein gleichzeitiger Schutz der empfindlichen Membranen erreicht. Durch die Schutzpapiere 13 und 14 werden in Fig. 3 über der Membranfläche 3 schmale Hohlräume 15 abgedeckt. Diese Hohlräume 15 können gegebenenfalls schon bei Herstellung der Klebehalterung mit Kontaktflüssigkeit bzw. Elektrolyt versehen werden. In der Fig. 3 ist der dem Meßweraufnehmer zugewandte Hohlraum bereits mit einem Elektrolyten 16 ausgefüllt.

Die Membran 3 nach Fig. 1 bis 3 ist sauerstoffdurchlässig und hat formelastische Eigenschaften. Als Materialien kommen bevorzugt Polypropylen, Polyäthylen oder Polytetrafluoräthylen in Frage. Die Stärke solcher üblicherweise verwendeten Membranen liegt zwischen 6 und 30 $\mu$m.

In der Fig. 4 ist mit der erfindungsgemäßen Klebehalterung ein Meßwertaufnehmer 20, z.B. eine Sauerstoffelektrode für transkutane Messungen, an einem Meßort appliziert. Eine solche Sauerstoffelektrode wird durch ein scheibenförmiges Trägerteil 21 mit Leitungsanschluß 22 und ebener ringförmiger Applikationsfläche 23 gebildet. Zentrisch im Trägerteil 21 sind Kathode und Anode für die polarographische Messung eingesetzt. Dabei ist die Anode als Hohlzylindertopf 24 mit einer leicht konvexen Grundfläche 25 ausgebildet, in der sich in einer kreisförmigen Ausnehmung 26 konzentrisch die drahtförmige Kathode 27 befindet. Auf der Grundfläche des Anodentopfes 24 ist ein Heizelement 28 zur Beheizung des Aufnehmers angeordnet. Weiterhin sind mit 29 bis 32 elektrische Leitungen für Elektroden und Heizung bezeichnet.

Es hat sich als zweckmäßig erwiesen, die Ausnehmung 2 der Klebehalterung nach Fig. 2 bis 3 etwas größer als den Durchmesser der Anodengrundfläche 25 zu wählen. Neben der notwendigen Fertigungstoleranz wird dadurch auch bei Anbringen der Klebehalterung am Aufnehmer gewährleistet, daß die Membran dicht abschließt.

Bei Applikation einer solchen Sauerstoffelektrode wird folgendermaßen verfahren: Ein Elektrolyt wird auf die Elektrodenfläche aufgebracht, mittels Lasche 12 wird das obere Schutzpapier 11 vorsichtig vom Klebering 7 abgezogen. Der Klebering 7 kann an einem Umfangsabschnitt beginnend an der Applikationsfläche des Aufnehmers, angeklebt werden. Durch weiteres Andrücken, beginnend von dem bereits fixierten Abschnitt aus, wird nun die gesamte Klebehalterung auf der Applikationsfläche aufgebracht. Die Bildung von Luftblasen im Elektrolyten zwischen Elektrodenfläche und Membran wird durch Herausdrücken der Luft zur offenen Seite vermieden. Gleichzeitig wird nach komplettem Ankleben des Ringes durch die leicht konvexe Form der Elektrodengrundfläche die formelastische Membran geringfügig vorgespannt.

Nach Abziehen des unteren Schutzpapiers 9 mittels Lasche 10 kann nun der Meßwertaufnehmer am Meßort appliziert werden.

Bei Verwendung einer Klebehalterung nach Fig. 3 ergibt sich noch eine weitere Vereinfachung, da das separate Aufbringen des Elektrolyten auf die Elektrodenfläche entfällt.

Nach Beendigung der Messung kann der Meßwertaufnehmer mittels Anfaßlaschen 6 bzw. 8 vom Meßort abgezogen werden. Es hat sich gezeigt, daß für unmittelbar nachfolgende Messungen häufig ein Totalersatz der Klebehalterung mit Membran nicht notwendig ist. In vielen Fällen braucht vielmehr lediglich die untere Klebefolie 5 mittels Anfaßlasche 6 abgezogen werden. Die obere Klebefolie 7 mit Membran 3 bleibt unter Einschluß des Elektrolyten am Meßwertaufnehmer fixiert. Die untere Klebefolie 5 wird in diesem Fall lediglich durch einen Klebering, wie er beispielsweise bei der Applikation von EKG-Elektroden eingeführt ist, ersetzt.

Insgesamt ergeben sich also bei Verwendung der erfindungsgemäßen Klebehalterungen erhebliche Vereinfachungen bei der Handhabung von transkutanen Sauerstoffelektroden.

Speziell für die Applikation von transkutanen Sauerstoffelektroden ist die Klebehalterung zweckmäßigerweise als geschlossener Ring mit Membran in der Ausnehmung des Ringes ausgebildet. In anderen Ausführungsformen der Erfindung kann die Klebehalterung auch einen Halbring mit U-förmiger oder sichelförmiger Klebefläche mit darin eingelassener Membran bilden. Es ist aber auch möglich, daß die Membran von der Klebefläche als Fahne weggeführt ist. In allen Fällen ist aber die Membran Teil der Klebehalterung.

**Patentansprüche**

1. Klebehalterung für Meßwertaufnehmer, die eine Membran benötigen, insbesondere für transkutane Sauerstoffelektroden, mit einem Klebeträger (1), der beidseitig wenigstens teilweise Klebeflächen aufweist, zum Ankleben an die Applikationsfläche (23) des Aufnehmers (20) einerseits und zum Ankleben des Klebeträgers (1) samt Aufnehmer (20) am Meßort andererseits, dadurch gekennzeichnet, daß der Klebeträger (1) als Ring aus zwei aufeinanderliegenden, je beidseitig klebenden Folien (5, 7) gebildet wird, zwischen denen die Membran (3) derart angeordnet ist, daß die Membran (3) die Ringausnehmung (2) im Klebeträger (1) abdeckt und selbst einen Teil der Klebehalterung für den Meßwertaufnehmer (20) bildet.

2. Klebehalterung nach Anspruch 1, dadurch gekennzeichnet, daß die Membran (3) sauerstoffdurchlässig ist.

3. Klebehalterung nach Anspruch 1, dadurch gekennzeichnet, daß die Membran (3) formelastisch ist.

4. Klebehalterung nach Anspruch 2, dadurch gekennzeichnet, daß die Membran (3) aus für größere Ionen und Moleküle undurchlässigem Material, wie Polypropylen, Polyäthylen, Polytetrafluoräthylen od. dgl., besteht.

5. Klebehalterung nach Anspruch 1, dadurch gekennzeichnet, daß der Klebeträger (1) wenigstens eine nicht klebende Anfaßlasche (4) aufweist.

6. Klebehalterung nach Anspruch 5, dadurch gekennzeichnet, daß jede Klebefolie (5, 7) des Klebeträgers (1) separate, nicht klebende Anfaßlaschen (6, 8) aufweist.

7. Klebehalterung nach Anspruch 6, dadurch gekennzeichnet, daß die dem Aufnehmer (20) zugewandte Anfaßlasche (8) länger als die der Applikationsfläche (18) zugewandte Anfaßlasche (6) ist.

8. Klebehalterung nach Anspruch 1, dadurch gekennzeichnet, daß der von den Folien (5, 7) gebildete Klebeträger (1) wenigstens auf den Klebeflächen mit je einem die Klebeflächen überdeckenden Schutzpapier (9, 11; 13, 14) versehen ist.

9. Klebehalterung nach Anspruch 8, dadurch gekennzeichnet, daß die Schutzpapiere (13, 14) die sauerstoffdurchlässige Membran (3) abdecken.

10. Klebehalterung nach Anspruch 9, dadurch gekennzeichnet, daß im Raum zwischen Membran (3) und dem Schutzpapier (14), welches die dem Aufnehmer (20) zugewandte Klebefolie (7) bedeckt, ein Elektrolyt (16) eingeschlossen ist.

11. Verwendung einer Klebehalterung nach einem der Ansprüche 8 bis 10 zur Applikation eines Aufnehmers (20) zur transkutanen Messung des Sauerstoffpartialdruckes, der wenigstens eine Meßelektrode (27) und wenigstens eine Gegenelektrode (24) aufweist, dadurch gekennzeichnet, daß der von den Klebefolien gebildete ringförmige Klebeträger (1) nach Abzug des ersten Schutzpapiers (11) unter Einschluß eines Elektrolyten (16) zwischen Membran (3) des Klebeträgers (1) und der Meßelektrode (27) sowie Gegenelektrode (24) bündig auf die Applikationsfläche (23) des Aufnehmers (20) geklebt wird, so daß sich aus Elektroden (24, 27), Elektrolyt (16) und Membran (3) eine komplette polarographische Meßzelle ergibt.

12. Verwendung einer Klebehalterung nach einem der Ansprüche 1 bis 10 zur Applikation eines Aufnehmers (20) zur transkutanen Messung des Sauerstoffpartialdruckes, der wenigstens eine Meßelektrode (27) und wenigstens eine Gegenelektrode (24) aufweist, dadurch gekennzeichnet, daß zwecks dichter Anlage der Membran (3) auf der mit dem Elektrolyten versehenen Meßelektrode (27) die formelastische Membran (3) durch Fixieren des Klebeträgers (1) auf der Applikationsfläche (23) des Meßgrößenaufnehmers (20) mit Vorspannung angebracht wird, wobei die Vorspannung der Membran (3) im wesentlichen durch eine leicht konvexe Ausbildung der Gegenelektrode (24) bewirkt wird.

**Revendications**

1. Fixation par collage pour des capteurs de valeurs de mesure, qui nécessitent la mise en oeuvre d'une membrane, en particulier pour des électrodes destinées à déterminer par voie transcutanée la teneur en oxygène du sang (électrode d'oxygène), du type comportant un support d'adhésif (1) qui est pourvu sur ses deux faces au moins de surfaces partielles de collage, pour, d'une part, le collage sur la surface d'application (23) du capteur (20) et pour, d'autre part, le collage du support de collage (1), y compris le capteur (20), à l'emplacement de la mesure, caractérisée par le fait que le support de l'adhésif (1) est constitué sous la forme d'un anneau de deux feuilles superposées (5, 7) et collantes sur leurs deux faces, et entre lesquelles la membrane (3) est disposée de telle manière qu'elle recouvre le trou (2) du support d'adhésif (1) et form elle-même une partie de la fixation collante pour le capteur de valeurs de mesure (20).

2. Fixation par collage selon la revendication 1, caractérisée par le fait que la membrane (3) est perméable à l'oxygène.

3. Fixation par collage selon la revendication 1, caractérisée par le fait que la membrane présente une élasticité de forme.

4. Fixation par collage selon la revendication 2, caractérisée par le fait que le membrane (3) est constituée par un matériau tel que le polypropylène, le polyéthylène, le polytétrafluoroéthylène ou un autre matériau similaire, imperméable pour des ions et des molécules de grande dimension.

5. Fixation par collage selon la revendication 1, caractérisée par le fait que le support d'adhésif (1) comporte au moins une patte de préhension (4) qui n'est pas collante.

6. Fixation par collage selon la revendication 5, caractérisée par le fait que chaque feuille collante (5, 7) du support de collage (1) comporte des pattes de préhension séparées (6, 8) non collantes.

7. Fixation par collage selon la revendication 6, caractérisée par le fait que la patte de préhension (8) qui est voisine du capteur (20) est plus longue que la patte de préhension (6) qui est voisine de la surface d'application (18).

8. Fixation par collage selon la revendication 1, caractérisée par le fait que le support de collage (1) qui est formé par les feuilles (5, 7), est pourvu, au moins sur les surfaces collantes, respectivement d'un papier de protection (9, 11; 13, 14) qui recouvre une surface collante.

9. Fixation par collage selon la revendication 8, caractérisée par le fait que les papiers de protection (13, 14) recouvrent la membrane (3) perméable à l'oxygène.

10. Fixation par collage selon la revendication 9, caractérisée par le fait que dans l'espace situé entre la membrane (3) et le papier de protection (14) qui recouvre la feuille collante (7) qui est voisine du capteur (20), est enfermé un électrolyte (16).

11. Application d'une fixation par collage selon l'une des revendications 8 à 10 pour l'application d'un capteur (20) pour la mesure, transcutanée, de la pression partielle de l'oxygène et comportant au moins une électrode de mesure (27) et au moins une contre-électrode (24), caractérisée par le fait que le support d'adhésif annulaire (1), formé par les feuilles collantes, est collé à plat sur la surface d'application (23) du capteur (20), après dégagement du premier papier de protection (1), avec emprisonnement d'un électrolyte (16) entre la membrane (3) du support d'adhésif (1) et l'électrode de mesure (27) de même que la contre-électrode (24), en sorte qu'il en résulte une cellule de mesure polarographique complète constituée par les électrodes (24, 27), l'électrolyte (16) et la membrane (3).

12. Application d'une fixation par collage selon l'une des revendications 1 à 10 pour l'application d'un capteur (20) pour la mesure, par voie transcutanée, de la pression partielle de l'oxygène, du type comportant au moins une électrode de mesure (27) et au moins une contre-électrode (24), caractérisée par le fait que pour une application étanche de la membrane (3) contre l'électrode de mesure (27) pourvue de l'électrolyte, la membrane (3), présentant une élasticité de forme, est mise en place avec une tension préalable, par fixation du support d'adhésif (1) sur la surface d'application (23) du capteur de grandeurs de mesure (20), la tension préalable de la membrane (3) étant essentiellement provoquée par une conformation légèrement convexe de la contre-électrode (24).

## Claims

1. An adhesive mounting for measured value pick-ups which require a membrane, in particular for transcutaneous oxygen electrodes, comprising an adhesive carrier (1), which at least in part has adhesive surfaces on both sides, for sticking on to the application surface (23) of the pick-up (20), on the one hand, and for sticking the adhesive carrier (1), together with the pick-up (20) to the measuring point, on the other hand, characterised in that the adhesive carrier (1) is in the form of a ring consisting of two superimposed foils (5, 7) which are adhesive on both sides, and between which the membrane (3) is arranged in such a way that the membrane (3) covers the central opening (2) of the adhesive carrier (1) and itself forms a part of the adhesive mounting for the measured value pick-up (20).

2. An adhesive mounting as claimed in Claim 1, characterised in that the membrane (3) is pervious to oxygen.

3. An adhesive mounting as claimed in Claim 1, characterised in that the membrane (3) is elastic.

4. An adhesive mounting as claimed in Claim 2, characterised in that the membrane (3) consists of a material, such as polypropylene, polyethylene, polytetrafluoroethylene, or the like, which is impervious to large ions and molecules.

5. An adhesive mounting as claimed in Claim 1, characterised in that the adhesive carrier (1) has at least one non-adhesive tongue (4) by which it can be handled.

6. An adhesive mounting as claimed in Claim 5, characterised in that each adhesive film (5, 7) of the adhesive carrier (1) has a separate non-adhesive handling tongue (6, 8).

7. An adhesive mounting as claimed in Claim 6, characterised in that the handling tongue (8) which faces towards the pick-up (20) is longer than the handling tongue (6) which faces towards the application surface (18).

8. An adhesive mounting as claimed in Claim 1, characterised in that at least on the adhesive surfaces, the adhesive carrier (1) which is formed by the films (5, 7) is provided with protecting papers (9, 11; 13, 14) which cover the respective adhesive surfaces.

9. An adhesive mounting as claimed in Claim 8, characterised in that the protecting papers (13, 14) cover the oxygen-pervious membrane (3).

10. An adhesive mounting as claimed in Claim 9, characterised in that an electrolyte (16) is housed in the space between the membrane (3) and the protective paper (14) which covers the adhesive film (7) facing the pick-up (20).

11. The use of an adhesive mounting as claimed in one of Claims 8 to 10 for the application of a pick-up (20) for the transcutaneous measurement of oxygen partial pressure, which pick-up (20) has at least one measuring electrode (27) and at least one counter-electrode (24), characterised in that, after the removal of the first protective paper (11), the annular adhesive carrier (1) which is formed by the adhesive films is stuck evenly to the application surface (23) of the pick-up (20), to enclose an electrolyte (16) between the membrane (3) of the adhesive carrier (1) and both the measuring electrode (27) and the counter-electrode (24), so that the electrodes (24, 27), the electrolyte (16) and the membrane (3) form a complete polarographic measuring cell.

12. The use of an adhesive support as claimed in one of Claims 1 to 10 for the application of a pick-up (20) for the transcutaneous measurement of oxygen partial pressure, which receive comprises at least one measuring electrode (27) and at least one counter-electrode (24), characterised in that to ensure a close contact of the membrane (3) with the measuring electrode (27) which is provided with electrolyte, the elastic membrane (3) is applied under stress by fixing the adhesive carrier (1) to the application surface (23) of the measured value pick-up (20), the stressing of the membrane (3) being essentially effected by means of a slightly convex formation of the counter-electrode (24).

0 008 460

FIG 1

FIG 2

FIG 3

FIG 4